# EUROPÄISCHE PATENTSCHRIFT

(11) **EP 2 557 941 B1**
(45) Veröffentlichungstag und Bekanntmachung des Hinweises auf die Patenterteilung: **18.02.2015**
(21) Anmeldenummer: 11714211.7
(22) Anmeldetag: 24.03.2011
(51) Int. Cl.: A23K 1/16, A23K 1/18, A61P 33/00, A61P 1/14, A61P 1/04, A61P 1/12, A23K 1/14

(54) **TIERFUTTERZUSATZ MIT ANTIMIKROBIELLER UND LEISTUNGSFÖRDERNDER WIRKUNG**
ANIMAL FEED ADDITIVE HAVING AN ANTIMICROBIAL AND GROWTH-PROMOTING EFFECT
ADDITIF D'ALIMENTATION ANIMALE AYANT UNE ACTION ANTI-MICROBIENNE ET TONIFIANTE

(30) Priorität: 15.04.2010 AT 6102010
(43) Veröffentlichungstag der Anmeldung: 20.02.2013
(73) Patentinhaber: Neufeld, Klaus, 2532 Heiligenkreuz (AT)
(72) Erfinder: Neufeld, Klaus, 2532 Heiligenkreuz (AT)
(74) Vertreter: Beer & Partner Patentanwälte KG
(86) Internationale Anmeldenummer: PCT/AT2011/000148
(87) Internationale Veröffentlichungsnummer: WO 2011/127496

(56) Entgegenhaltungen:
- WO-A2-02/21933
- CN-A- 1 124 583
- CN-A- 101 543 261
- CN-A- 101 543 263
- CN-A- 101 579 043

## Beschreibung

Die Erfindung betrifft einen Tierfutterzusatz mit antimikrobieller, leistungsfördernder und appetitfördernder Wirkung und dessen Verwendung zur Herstellung eines Tierfuttermittels für Nutztiere.

Sogenannte Leistungsförderer wurden in der Nutztierfütterung weltweit über einen langen Zeitraum hinweg angewendet. Die Hauptgruppe der Leistungsförderer waren antibiotische und chemische Leistungsförderer, wie etwa Zink-Bacitracin, Flavophospholipol, Virginiamycin, Tylosin-Phosphat, Avoparcin, Olaquindox und Monensin-Natrium, welche auch in Österreich als leistungsfördernde Futterzusatzzustoffe zugelassen waren. Weiters gibt es seit einigen Jahren sogenannte Probiotica oder mikrobiologische Leistungsförderer.

Der Zweck aller Leistungsförderer ist es, die Wachstumsintensität der Nutztiere zu erhöhen.

Antibiotische und chemische Leistungsförderer sind allerdings in den letzten Jahren, vor allem beim Konsumenten in Verruf geraten und wurden im Jahre 2006 in der gesamten Europäischen Union als Futterzusatzstoffe verboten. International wird die Verwendung der antibiotischen und chemischen Leistungsförderer eingeschränkt oder verboten.

Mikrobiologische Leistungsförderer (Probiotica) wurden mit dem Ziel entwickelt, eine ökonomische Tierproduktion sicher zu stellen und gleichzeitig dem Wunsch des Konsumenten nach einer natürlichen Tierernährung zu entsprechen. Jedoch erfüllen Probiotica nicht ganz die ökonomischen Erwartungen der modernen Landwirtschaft, die zu einem effizienten Produktionsprozess gezwungen ist.

Weiters wurden pflanzliche Substanzen mit antimikrobiellen Eigenschaften in vielen Ländern in der tierischen Produktion etabliert. Nicht zuletzt, weil in zahlreichen Ländern die Verwendung chemischer und antibiotischer Leistungsförderer verboten wurde. Aufgrund des pflanzlichen Ursprungs, kostspieliger Extraktionsverfahren und der Volatilität mancher dieser Produkte sind viele dieser Substanzen aus Sicht eines ökonomischen Produktionsprozesses für die Verwendung in der Landwirtschaft nicht rentabel.

In den Patenten Nrn. AT 403873B, EP 0581926B1, EP 1317187B1, EP 1317188B1 werden solche pflanzlichen Leistungsförderer basierend auf Isochinolinalkaloiden beschrieben. Diese Produkte haben in den letzten Jahren weltweit eine großflächige Verbreitung gefunden. Aufgrund der Entwicklung in der landwirtschaftlichen Produktion und einem kontinuierlichen Ansteigen der Futtermittelpreise ist es jedoch notwendig, auch diese Produkte für den Markt ökonomischer bzw. in der Wirkung noch effizienter zu gestalten.

Hier will die Erfindung Abhilfe schaffen.

Erfindungsgemäß wird ein Tierfutterzusatz mit antimikrobieller, leistungsfördernder und appetitfördernder Wirkung vorgeschlagen, welcher dadurch gekennzeichnet ist, dass er ein alkaloidhältiges Pflanzenextrakt beziehungsweise Pflanzenmaterial aus Pflanzen der Gattung Macleaya in Kombination mit einem Extrakt bzw. Material aus Pflanzen der Gattung Magnolia enthält.

Unter den Begriff "Pflanzenmaterial" fallen sowohl die getrocknete Ganzpflanze als auch Teile davon, wie etwa Blätter, Blüten, Früchte, Wurzeln, Rhizome oder die Rinde. Diese Substanzen werden vorwiegend pulverförmig oder in granulierter Form eingesetzt. Freilich ist es auch möglich, das Pflanzenmaterial zu extrahieren, um anschließend das flüssige Extrakt selbst oder das eingedampfte Extrakt zu verwenden.

Die erfindungsgemäß eingesetzten Wirkstoffkomponenten bewirken einen Synergieeffekt hinsichtlich antimikrobieller Wirkung bei gleichzeitiger Leistungsförderung und / oder Appetitförderung der untersuchten Kontrolltiere. Dieser Synergieeffekt ist auf das Zusammenwirken der Alkaloide, insbesondere Isochinolinalkaloide, mit den Wirkstoffen aus der Magnolia, wie Magnolol und Honokiol zurückzuführen.

Weitere vorteilhafte Ausgestaltungen des erfindungsgemäßen Tierfutterzusatzes sind gemäß Unteransprüche 2 bis 10 offenbart.

Die Erfindung betrifft weiters ein Tierfuttermittel, welches dadurch gekennzeichnet ist, dass der erfindungsgemäße Tierfutterzusatz in einer Menge von 0,001 g/t bis 10 kg/t Futtermittel vorliegt.

Die Erfindung betrifft weiters die Verwendung des erfindungsgemäßen Verfahrens zur Herstellung eines Tierfuttermittels für Nutztiere, welches dadurch gekennzeichnet ist, dass den Nutztieren das erfindungsgemäße Tierfuttermittel verfüttert wird, welches den erfindungsgemäßen Tierfutterzusatz in einer Menge von 0,0001mg bis 350mg pro kg Lebendgewicht pro Tag enthält.

Die Erfindung betrifft weiters die Verwendung des erfindungsgemäßen Tierfutterzusatzes zur Herstellung eines Tierfuttermittels für die Behandlung oder vorbeugende Behandlung von Tieren gegen krankheitserregende Mikroben.

Die Erfindung wird nunmehr anhand von Versuchen bzw. vergleichenden Testreihen näher erläutert.

In einem in vitro Versuch konnte mittels Plattenhemmstofftest nachgewiesen werden, dass eine Kombination von einem Pflanzenextrakt aus Macleaya cordata und einem Pflanzenextrakt aus Magnolia spp. bspw. der Magnolia acuminata, Magnolia biondii, Magnolia denudata, Magnolia grandiflora, Magnolia kobus, Magnolia obovata, Magnolia officinalis, Magnolia sprengeri, Magnolia tripetala, Magnolia virginiana, Magnolia glauca im Verhältnis 1:1 zu einer deutlichen Verbesserung der Hemmwirkung gegenüber Staphylococcus aureus führte, als dies bei der gleichen Gewichtsmenge eines Extraktes aus Macleaya cordata bzw. eines Extraktes aus Magnolia spp. der Fall ist.

Zur Durchführung dieser Testreihen werden die Wirkstoffe der Macleaya cordata vorzugsweise durch Extraktion aus deren Früchten gewonnen. Die Früchte werden bei 50°C bis zu einem Trockensubstanzgehalt von mindestens 91% getrocknet und anschließend vermahlen. Das so gewonnene Pulver wird in einem Perkolator mit 0,1 molarer Salzsäurelösung als Extraktionsmittel bei einer Temperatur von 100°C extrahiert. Anschließend wird das Extrakt eingedampft, in Ethanol aufgenommen und auf den gewünschten Wirkstoffgehalt eingestellt.

Die Wirkstoffe aus Magnolia officinalis werden vorzugsweise aus der Rinde mittels superkritischer CO₂ Extraktion gewonnen. Das Rohmaterial wird gewaschen und bei 50°C bis zu einem Trockensubstanzgehalt von mindestens 91% getrocknet und anschließend zerkleinert. Im Extraktor wird das so aufbereitete Rohmaterial mit superkritischem CO₂ als Extraktionsmittel bei einer Durchflussrate von 1.200 bis 1.400 Liter pro Stunde für einen Zeitraum von 3,5 Stunden bei einem Druck von 25 bis 30 MPa und einer Temperatur von 35 bis 40°C extrahiert. Der CO₂ Extrakt wird anschließend in Ethanol aufgenommen und auf den gewünschten Wirkstoffgehalt eingestellt.

Eine beispielhafte Menge von 25 bis 50mg an gewonnenem, erfindungsgemäßem Tierfutterzusatzstoff weist nunmehr einen Sanguinaringehalt von 1,5% auf und kann in ein übliches Fertigfutter eingemischt werden. Es ist jedoch auch möglich, den erfindungsgemäßen Tierfutterzusatzstoff mit einem niedrigeren Wirkstoffgehalt als 1,5% bereitzustellen, wenn die Wirkstoffe aus nicht extrahiertem Pflanzenmaterial, z.B. getrockneten Blättern von Macleaya cordata, gewonnen werden. In derartigen Fällen muss die Dosierung allerdings wesentlich höher sein. Andererseits fallen auch sogenannte Vormischungen und Ergänzungsfuttermittel unter den Begriff Futtermittel. Vormischungen und Ergänzungsfuttermittel sind Futterkonzentrate, die mit anderen Futterkomponenten vermischt werden, um Fertigfutter herzustellen. Vormischungen und Ergänzungsfuttermittel enthalten daher den erfindungsgemäßen Tierfutterzusatz in wesentlich höherer Konzentration, da sie dazu gedacht sind, stark verdünnt zu werden. Somit lässt sich beispielsweise eine Dosierung von 10kg/t einstellen.

Eine niedrige Dosierung von 1 mg/t wird beispielsweise dann eingestellt, wenn der erfindungsgemäße Tierfutterzusatzstoff ein Extrakt mit hohem Wirkstoffanteil ist.

Im folgenden vitro Versuch werden jedenfalls Futtermittel ausgetestet, welche die Wirkstoffkombination aus Macleaya cordata und Magnolia spp. im Verhältnis 1:1 aufweisen.

Die konkreten Messwerte zu diesen vitro Versuch sind folgender Aufstellung zu entnehmen:

| Wirkstoffextrakte | Macleaya cordata | Magnolia spp. | Macleaya cordata + Magnolia spp. 1:1 |
|---|---|---|---|
| Breite des Hemmhofes | 6 mm | 5 mm | 8-10 mm |
| Keim | Staphylococcus aureus | | |

Im Rahmen von verschiedenen Fütterungsversuchen konnte mit einer Kombination aus einem Extrakt von Macleaya cordata und einem Extrakt von Magnolia spp. im Vergleich zu einem herkömmlichen Futtermittelzusatz aus Macleaya cordata alleine oder Magnolia spp. alleine eine deutliche Verbesserung der Wachstumsleistung erzielt werden.

**Tabelle 1**

| | Kontrollgruppe | Macleaya cordata | Macleaya cordata + Magnolia spp. 1:1 |
|---|---|---|---|
| Einmischrate des Tierfutterzusatzes | - | 30 ppm | 30ppm |
| Anzahl der Mastschweine | 80 | 80 | 80 |
| Versuchsperiode | 30-100kg | | |
| Ø Tageszunahme (g) Gewichtsbereich 30-100kg | 831 | 868 | 880 |
| Ø Tageszunahme (g) Gewichtsbereich 30-53kg | 775 | 788 | 801 |
| Ø Tageszunahme (g) Gewichtsbereich 53-100kg | 868 | 914 | 925 |
| Ø tägliche Futteraufnahme (kg) Gewichtsbereich 30-100kg | 2,30 | 2,33 | 2,33 |
| Ø tägliche Futteraufnahme (kg) Gewichtsbereich 53-100kg | 2,57 | 2,66 | 2,68 |
| Futterverwertung 30-100kg | 2,76 | 2,69 | 2,65 |
| Futterverwertung 30-53kg | 2,28 | 2,22 | 2,20 |
| Futterverwertung 53-100kg | 2,99 | 2,91 | 2,90 |
| Magerfleischanteil | 59,5 | 59,9 | 60,0 |

Aus den in Tabelle 1 angegebenen Werten ist zu erkennen, dass bei Verfütterung eines Materials, welches ausschließlich aus Macleaya cordata gewonnen oder aus dieser Pflanze extrahiert wurde, eine im Schnitt um 12 g geringere tägliche Gewichtszunahme über die gesamte Mastperiode zu bemerken war, als dies bei einer Ausführungsform des erfindungsgemäßen Tierfutterzusatzes aus Macleaya cordata und Magnolia spp. im Verhältnis 1:1 der Fall ist.

Weiters ist aus Tabelle 1 ersichtlich, dass die Futteraufnahme in allen Mastbereichen im Vergleich zur Kontrollgruppe erhöht ist. Bei Hochleistungsmastschweinen mit durchschnittlichen Tageszunahmen von mehr als 830g (auch schon in der Kontrollgruppe ohne leistungsfördernden Futterzusatz!) handelt es sich um Spitzenleistungen mit extremer Futteraufnahme. In diesen Leistungsbereichen ist jede weitere Steigerung als Erfolg zu werten.

**Tabelle 2**

| | Kontrollgruppe | Magnolia spp. | Macleaya cordata + Magnolia spp. 1:1 |
|---|---|---|---|
| Einmischrate des Tierfutterzusatzes | -- | 50ppm | 50ppm |
| Anzahl der Mastschweine | 48 | 47 | 50 |
| Versuchsperiode | 30-100kg | | |
| Ø Tageszunahme (g) Gewichtsbereich 30-100kg | 798 | 819 | 846 |
| Ø tägliche Futteraufnahme (kg) Gewichtsbereich 30-100kg | 2,24 | 2,28 | 2,32 |
| Futterverwertung 30-100kg | 2,81 | 2,78 | 2,74 |

Bei alleiniger Verfütterung von Extrakten bzw. Materialien der Magnolia spp. wurde eine um 27 g geringere tägliche Gewichtszunahme erzielt, als dies im Vergleich zum erfindungsgemäßen Tierfutterzusatz vermerkt wurde.

Weiters konnte in diesem Versuch mit dem erfindungsgemäßen Tierfutterzusatz eine um 80g höhere tägliche Futteraufnahme gegenüber der Kontrollgruppe verzeichnet werden.

Ähnliche Werte konnten im Zuge von Versuchsreihen, welche die Kontrolle der Gewichtszunahme von Ferkeln zum Gegenstand hatten, siehe dazu Werte aus Tabelle 3 und 4, erzielt werden.

**Tabelle 3**

| | Kontrollgruppe | Macleaya cordata | Macleaya cordata + Magnolia spp. 1:1 |
|---|---|---|---|
| Einmischrate des Tierfutterzusatzes | -- | 25 ppm | 25 ppm |
| Anzahl der Ferkel | 49 | 49 | 49 |
| Versuchsperiode (Alter) | 40-60 Tage | | |
| Aufzuchtphase | 12-32 Tage nach Absetzen | | |
| Ø Körpergewicht zu Versuchsbeginn (kg) | 13,5 | 13,2 | 13,2 |
| Ø Körpergewicht zu Versuchsende (kg) | 25,6 | 25,7 | 26,0 |
| Ø Tageszunahme (g) | 607 | 624 | 640 |
| Ø tägliche Futteraufnahme (g) | 895 | 902 | 907 |
| Futterverwertung (kg Futteraufnahme / kg Zunahme) | 1,47 | 1,45 | 1,42 |

**Tabelle 4**

| | Kontrollgruppe | Magnolia spp. | Macleaya cordata + Magnolia spp. 1:1 |
|---|---|---|---|
| Einmischrate des Tierfutterzusatzes | -- | 25ppm | 25 ppm |
| Anzahl der Ferkel | 49 | 49 | 49 |
| Versuchsperiode (Alter) | 40-60 Tage | | |
| Aufzuchtphase | 12-32 Tage nach Absetzen | | |
| Ø Körpergewicht zu Versuchsbeginn (kg) | 13,1 | 13,3 | 13,2 |
| Ø Körpergewicht zu Versuchsende (kg) | 25,4 | 26,1 | 26,2 |
| Ø Tageszunahme (g) | 615 | 640 | 650 |
| Ø tägliche Futteraufnahme (g) | 916 | 928 | 922 |
| Futterverwertung (kg Futteraufnahme / kg Zunahme) · | 1,49 | 1,45 | 1,42 |

In allen angeführten Versuchen ist also die Futteraufnahme im Vergleich zur Kontrollgruppe erhöht. Ein Vergleich zu jener Versuchsgruppe, die mit anderen, ebenfalls als Appetitförderer bekannten Futterzusätzen versorgt wurde, fällt ebenfalls überwiegend positiv aus. Dazu ist zu bemerken, dass es im Bereich von Fütterungsversuchen durchaus normal ist, dass nicht alle Wirkungen eines Zusatzes in allen durchgeführten Versuchen in gleichem Ausmaß messbar sind, sondern dass es durchaus zu biologischen Schwankungen kommen kann.

Bei der Verfütterung des erfindungsgemäßen Tierfutterzusatzes konnte nicht nur eine Verbesserung der Gewichtszunahme verzeichnet werden, die über den Werten herkömmlich bekannter Leistungsförderer lag, sondern auch eine Verbesserung des Gesundheitszustandes, der sich in einer niedrigeren krankheitsbedingten Ausfallsrate widerspiegelt.

Nekrotisierende Enteritis, ausgelöst durch Clostridium perfringens stellt nämlich beim Geflügel, insbesondere bei Legehennen, ein großes wirtschaftliches Problem dar. Die Symptome sind eine chronische Enteritis, verringerte Futteraufnahme, gesteigerte Wasseraufnahme, zu feuchter Kot, Veränderungen des Federkleides, Federpicken und Federfressen, Gewichtsabnahme, verringerte Legeleistung mit einer gesteigerten Varianz des Eigewichts, verkürzte Nutzungsdauer, Infektanfälligkeit sowie erhöhte Mortalität aufgrund von Erschöpfung oder Sekundärinfektionen, wie etwa E. coli Infektionen.

Die Krankheit erweist sich als langwierig und therapieresistent. Die Verabreichung von Antibiotika ist problematisch, da die Krankheitssymptome nach Absetzen der Therapie wiederkommen und eine Antibiotikatherapie aufgrund der geltenden Absetzfristen einen hohen ökonomischen Schaden verursacht. Eier können während der Therapie und während der Absetzfrist nicht verwertet werden, aufgrund der kurzen Mastdauer von Broilern (Masthühnern) können diese nicht therapiert werden. Andere mögliche Therapieansätze, wie Veränderung der Futterzusammensetzung, Anpassung des Rohfaser- oder Rohproteingehaltes im Futter, Verabreichung essentieller Aminosäuren oder Fettsäuren, Anpassung des Vitamin- oder Mineralstoffgehaltes im Futter, Zusatz von organischen Säuren etc., haben sich als ineffizient erwiesen.

In einem Legehennenbetrieb, in dem die oben beschriebenen Symptome bereits seit über zwei Jahren in verschiedenen Herden beobachtet wurden, wurde einer neu erkrankten Herde, ca. 5 Wochen nach Auftreten der ersten Symptome, die erfindungsgemäße Substanz in einer Dosierung von 100ppm (100g pro Tonne Futter) verabreicht. Damit wurde eine nachhaltige Besserung erzielt, die Herde wurde völlig symptomfrei und der Betrieb konnte somit wieder unter ökonomischen Gesichtspunkten produzieren.

Folgende, in Tabelle 5 angeführten Versuche belegen diese Ergebnisse. Dabei wurde der erfindungsgemäße Tierfutterzusatz auch im Vergleich mit bekannten antibiotischen Leistungsförderern getestet.

**Tabelle 5**

| | antibiotischer Leistungsförderer | Macleaya cordata | Macleaya cordata + Magnolia spp. 1:1 |
|---|---|---|---|
| Einmischrate des Tierfutterzusatzes | 15 ppm | 50 ppm | 30ppm |
| Anzahl der Broiler | 89.500 | 89.500 | 90.100 |
| Versuchsperiode (Tage) | 36 | 36 | 36 |
| Ø Tageszunahme (g) | 43,2 | 43,6 | 43,5 |
| Futterverwertung | 1,81 | 1,78 | 1,78 |
| Ausfälle (% der Broiler) | 7,4 | 4,2 | 3,9 |

**Tabelle 6**

| | antibiotischer Leistungsförderer | Magnolia spp. | Macleaya cordata + Magnolia spp. 1:1 |
|---|---|---|---|
| Einmischrate des Tierfutterzusatzes | 15ppm | 50ppm | 30ppm |
| Anzahl der Broiler | 43.200 | 41.500 | 42.000 |
| Versuchsperiode (Tage) | 35 | 35 | 35 |
| Ø Tageszunahme (g) | 44,1 | 43,8 | 44,3 |
| Futterverwertung | 1,79 | 1,83 | 1,81 |
| Ausfälle (% der Broiler) | 5,8 | 4,9 | 3,8 |

So lässt sich an den in Tabellen gezeigten Werten nachvollziehen, dass bei an sich bekannten antibiotischen Leistungsförderern immerhin Ausfälle von 7,4 % vermerkt werden konnten.

Obzwar bei Verfütterung von Macleaya cordata eine zusätzliche Reduzierung der Ausfälle erzielt wird, ist zweifelsfrei eine weitere Reduktion der Ausfälle wie in Tabellen 5 und 6 angeführt, ausschließlich auf die kombinatorische Wirkung von Macleaya cordata und Magnolia spp. zurückzuführen.

Ähnliche Ergebnisse konnten auch bei eierlegenden Nutztieren in Hinblick auf Anzahl der gelegten Eier, Qualitätssortierung der Eier, Futterverwertung und Ausfälle der Tiere beobachtet werden. Ein ebensolcher Effekt wurde auch bei Fischen in Hinblick auf Wachstumsintensität, Futteraufnahme und Ausfallmenge verzeichnet.

Ähnlich positive Ergebnisse wurden an Absetzferkeln und Mastschweinen beobachtet.

Bei Absetzferkeln und Mastschweinen treten oftmals die Durchfallerkrankungen Dysenterie, ausgelöst durch Brachyspira hyodysenteriae, sowie PIA (Porcine Intestinale Adenomatose), ausgelöst durch Lawsonia intracellularis, gehäuft auf. Diese Erkrankungen zeichnen sich durch ein Durchfallgeschehen mit grauem und wässrigem Kot aus. Die ökonomischen Schäden dieser Erkrankungen liegen in den Mehrausgaben für Therapie, verminderte Futteraufnahme, schlechtes Wachstum, Anfälligkeit für Sekundärkrankheiten und erhöhte Mortalität. Als Therapie eignet sich eine Antibiotikamedikation über das Futter über einen Zeitraum von 3 bis 4 Wochen mit parallel laufendem Hygieneprogramm. Aus veterinärmedizinischer und seuchenhygienischer Sicht ist eine derartige regelmäßige Antibiotikatherapie aufgrund der bekannten Resistenzproblematik mit Skepsis zu betrachten. Für den landwirtschaftlichen Betrieb bedeutet es eine erhebliche Kostenbelastung.

In mehreren Problembetrieben mit gehäuftem Auftreten von Dysenterie und PIA konnte mit dem erfindungsgemäßen Tierfutterzusatz in einer Dosierung von 60ppm (60 g pro Tonne Fertigfutter) eine nachhaltige Besserung der Krankheitssymptome und eine signifikante Reduktion der Ausfälle (Mortalität) erreicht werden.

Es konnte somit nachgewiesen werden, dass mit einer Kombination aus Pflanzenmaterial bzw. Extrakten der beiden Pflanzenarten Macleaya sowie Magnolia eine neuartige antimikrobiell wirksame Substanzkombination mit besonders günstigen Eigenschaften im Bereich der tierischen Produktion entwickelt wurde.

## Patentansprüche

1. Tierfutterzusatz mit antimikrobieller, leistungsfördernder und appetitfördernder Wirkung, **dadurch gekennzeichnet, dass** er ein alkaloidhaltiges Extrakt beziehungsweise Material aus Pflanzen der Gattung Macleaya in Kombination mit einem Extrakt bzw. Material aus Pflanzen der Gattung Magnolia enthält.

2. Tierfutterzusatz nach Anspruch 1, **dadurch gekennzeichnet, dass** das Extrakt bzw. Material aus Pflanzen der Gattung Magnolia Magnolol und / oder Honokiol enthält.

3. Tierfutterzusatz nach Anspruch 1 oder 2, **dadurch gekennzeichnet, dass** das Extrakt bzw. Material aus Pflanzen der Gattung Magnolia, nämlich aus Magnolia spp. wie Magnolia acuminata, Magnolia biondii, Magnolia denudata, Magnolia grandiflora, Magnolia kobus, Magnolia obovata, Magnolia officinalis, Magnolia sprengeri, Magnolia tripetala, Magnolia virginiana, Magnolia glauca, gewonnen wird.

4. Tierfutterzusatz nach einem der Ansprüche 1 bis 3, **dadurch gekennzeichnet, dass** er in gleichen Anteilen Extrakte bzw. Materialien der Macleaya cordata und der Magnolia spp. enthält.

5. Tierfutterzusatz nach einem der Ansprüche 1 bis 3, **dadurch gekennzeichnet, dass** er in Anteilen von 1:100 bis 100:1 Extrakte bzw. Materialien der Macleaya cordata und der Magnolia spp. enthält.

6. Tierfuttermittel umfassend einen Tierfutterzusatz nach einem der Ansprüche 1 bis 5, **dadurch gekennzeichnet, dass** der Tierfutterzusatz in einer Menge von 0,001g/t bis 10kg/t Futtermittel vorliegt.

7. Verwendung eines Tierfutterzusatzes nach einem der Ansprüche 1 bis 5 zur Herstellung eines Tierfuttermittels für Nutztiere, **dadurch gekennzeichnet, dass** der Tierfutterzusatz pro Tag in einer Menge von 0,0001mg bis 350mg pro kg Lebendgewicht eingesetzt wird.

8. Verwendung eines Tierfutterzusatzes nach einem der Ansprüche 1 bis 5 zur Herstellung eines Tierfuttermittels für die Behandlung oder vorbeugende Behandlung von Tieren gegen krankheitserregende Mikroben.

9. Verwendung eines Tierfutterzusatzes nach einem der Ansprüche 1 bis 5 zur Herstellung eines Tierfuttermittels für die Behandlung von Durchfallerkrankungen, wie insbesondere Dysenterie und Porcine Intestinale Adenomatose (PIA).

10. Verwendung eines Tierfutterzusatzes nach einem der Ansprüche 1 bis 5 zur Herstellung eines Tierfuttermittels für die Behandlung von nekrotisierender Enteritis.

## Claims

1. An animal feed additive having an antimicrobial, growth-promoting and appetite-promoting effect, **characterised in that** it contains an alkaloid-containing extract or material from plants of the Macleaya genus in combination with an extract or material from plants of the Magnolia genus.

2. The animal feed additive according to claim 1, **characterised in that** the extract or material from plants of the Magnolia genus contains Magnolol and/or Honokiol.

3. The animal feed additive according to claim 1 or 2, **characterised in that** the extract or material from plants of the Magnolia genus is obtained specifically from Magnolia spp., such as Magnolia acuminate, magnolia biondii, Magnolia denudate, Magnolia grandiflora, Magnolia kobus, Magnolia obovata, Magnolia officinalis, Magnolia sprengeri, Magnolia tripetala, Magnolia virginiana, Magnolia glauca.

4. The animal feed additive according to one of claims 1 to 3, **characterised in that** it contains extracts or materials of Macleaya cordata and of Magnolia spp. in equal proportions.

5. The animal feed additive according to one of claims 1 to 3, **characterised in that** it contains extracts or materials of Macleaya cordata and Magnolia spp. in proportions from 1:100 to 100:1.

6. An animal feed comprising an animal feed additive according to one of claims 1 to 5, **characterised in that** the animal feed additive is present in a quantity from 0.001 g/t to 10 kg/t of feed.

7. Use of an animal feed additive according to one of claims 1 to 5 for producing an animal feed for livestock, **characterised in that** the animal feed additive is used per day in a quantity from 0.0001 mg to 350 mg per kg of live weight.

8. Use of an animal feed additive according to one of claims 1 to 5 for producing an animal feed for the treatment or preventative treatment of animals against pathogenic microbes.

9. Use of an animal feed additive according to one of claims 1 to 5 for producing an animal feed for the treatment of diarrhoea disorders, in particular such as dysentery and porcine intestinal adenomatosis (PIA).

10. Use of an animal feed additive according to one of claims 1 to 5 for producing an animal feed for the treatment of necrotising enteritis.

## Revendications

1. Additif pour l'alimentation animale, ayant un effet antimicrobien, stimulateur des performances et de l'appétit, **caractérisé en ce qu'**il contient un extrait, respectivement de la matière contenant des alcaloïdes, issu(e) de plantes de l'espèce Macleaya en association avec un extrait ou de la matière issue de plantes de l'espèce Magnolia.

2. Additif pour l'alimentation animale selon la revendication 1, **caractérisé en ce que** l'extrait ou la matière issu(e) de plantes de l'espèce Magnolia contient du magnolol et/ou de l'honokiol.

3. Additif pour l'alimentation animale selon la revendication 1 ou 2, **caractérisé en ce que** l'extrait ou la matière issu(e) de plantes de l'espèce Magnolia est obtenu(e) à partir de Magnolia spp., comme le Magnolia acuminata, le Magnolia biondii, le Magnolia denudata, le Magnolia grandiflora, le Magnolia kobus, le Magnolia obovata, le Magnolia officinalis, le Magnolia sprengeri, le Magnolia tripetala, le Magnolia virginiana, le Magnolia glauca.

4. Additif pour l'alimentation animale selon l'une quelconque des revendications 1 à 3, **caractérisé en ce qu'**il contient dans des parts identiques des extraits ou des matières de la Macleaya cordata et du Magnolia spp.

5. Additif pour l'alimentation animale selon l'une quelconque des revendications 1 à 3, **caractérisé en ce qu'**il contient des extraits ou des matières de la Macleaya cordata et du Magnolia spp dans des parts comprises entre 1:100 et 100:1.

6. Aliment pour animaux comprenant un additif pour l'alimentation animale selon l'une quelconque des revendications 1 à 5, **caractérisé en ce que** l'additif pour l'alimentation animale est présent dans une quantité de 0,001g/t à 10kg/t.

7. Utilisation d'un additif pour l'alimentation animale selon l'une quelconque des revendications 1 à 5 pour la fabrication d'un aliment pour animaux destinés à du bétail, **caractérisé en ce que** l'additif pour alimentation animale est mis en oeuvre dans une quantité de 0,0001 mg à 350 mg par kg de poids vif par jour.

8. Utilisation d'un additif pour l'alimentation animale selon l'une quelconque des revendications 1 à 5 pour la fabrication d'un aliment pour animaux destiné au traitement ou à la prévention des animaux contre des microbes pathogènes.

9. Utilisation d'un additif pour l'alimentation animale selon l'une quelconque des revendications 1 à 5 pour la fabrication d'un aliment pour animaux destiné au traitement de maladies diarrhéiques, comme notamment la dysenterie et l'adénomatose intestinale porcine (PIA).

10. Utilisation d'un additif pour l'alimentation animale selon l'une quelconque des revendications 1 à 5 pour fabriquer un aliment pour animaux pour le traitement de l'entérite nécrosante.
